Europäisches Patentamt

European Patent Office

Office européen des brevets

(11) Publication number: **0 369 041 B1**

(12) **EUROPEAN PATENT SPECIFICATION**

(45) Date of publication of patent specification: **31.03.93**  (51) Int. Cl.⁵: **C12Q 1/04**, C12Q 1/30, C12N 1/06

(21) Application number: **88118965.8**

(22) Date of filing: **14.11.88**

(54) Method, reagent mixture and kit for determining the presence of bacterial or somatic cells in urine.

| | |
|---|---|
| (43) Date of publication of application:<br>**23.05.90 Bulletin 90/21** | (73) Proprietor: **YISSUM RESEARCH DEVELOP-<br>MENT COMPANY OF THE HEBREW UNIVER-<br>SITY OF JERUSALEM<br>46 Jabotinsky Street<br>Jerusalem, 92 182(IL)** |
| (45) Publication of the grant of the patent:<br>**31.03.93 Bulletin 93/13** | |
| | (72) Inventor: **Citri, Nathan<br>8, Hahayl St.<br>Jerusael 97 981(IL)** |
| (84) Designated Contracting States:<br>**DE ES FR GB IT SE** | |
| (56) References cited:<br>**EP-A- 0 145 233<br>GB-A- 2 202 049<br>US-A- 3 764 479** | (74) Representative: **Brown, John David et al<br>FORRESTER & BOEHMERT Franz-<br>Joseph-Strasse 38<br>W-8000 München 40 (DE)** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.5x/3.0.1)

EP 0 369 041 B1

## Description

The present invention relates to a method for detecting the presence of cells in a urine sample. More particularly, the present invention provides a highly sensitive method and test kit for rapid detection of catalase in urine which is an indication for the presence of bacterial or somatic cells in the urine.

As is known, clean, healthy urine has no catalase activity. The presence of catalase indicates that the urine is contaminated or colonized by bacteria or that a pathological condition exists that has to be investigated. The common pathological conditions indicated by catalase activity in urine include significant asymptomatic bacteriuria, pyuria, hematuria and tissue damage anywhere from the kidney down to the urinary tract.

The diagnosis of any of the above conditions is based on well established laboratory procedures which must continue to provide the sole, reliable basis for a positive diagnosis. However, these procedures, especially those involving incubation of the urine samples, are rather time-consuming and costly and require the services of trained laboratory personnel. Consequently, there exists a need for methods designed for fast screening of a large number of urine samples which would be suitable, inter alia, for surveying a presumably healthy population, e.g., in schools and other institutions, where screening programs for asymptomatic bacteriuria are occasionally conducted. It may also serve as a preliminary screening of a large number of urines received for routine examination in large diagnostic laboratories, so as to discard healthy urines that need not be further investigated for any of the above mentioned pathological conditions. Specimens found to be negative by such a preliminary test need not be further investigated for significant bacteriuria, hematuria, pyuria or urinary tract tissue damage.

It is the object of the present invention to satisfy the above discussed need, i.e. to provide a simple, rapid, direct and reliable method for testing urine samples for the presence of bacteria or somatic cells, which method would completely obviate the need for incubation and would require no preparation, skill or special equipment, so that the test could be carried out in a matter of minutes in the physician's office or by the patient himself. The advantage of saving time, labor and cost of materials is obvious.

Israel Patent Specification No. 36496 describes and claims a method of testing for the presence of organic cells in a liquid which comprises the steps of disrupting said cells in a sample of the liquid by the combined action of a protease and detergent compatible therewith, whereby undergraded desoxyribonucleic acid (DNA) and active enzymes are realised, and thereafter testing said sample for the presence of DNA and/or of a released enzyme.

In preferred embodiments of said patent the liquid is urine and the cells are bacterial cells, and the released enzyme is catalase which is tested for by its reaction with $H_2O_2$ to release gaseous oxygen with resultant formation of foam. In this test, the assay of catalase activity follows the treatment of the specimen with a cell disrupting preparation. The treatment is intended to remove possible accessibility barriers between the catalase present inside the cells and its substrate. The presence of such barriers will slow down the catalytic reaction and thus interfere with the detection of catalase. Disruption of cells is thus intended for making the total catalase content of the specimen instantly detectable.

As described and explained in said patent, although disruption of bacterial cells can be accomplished by several well-known procedures, none of these is acceptable for the present purpose. The available methods are usually laborious and time-consuming, or else not generally effective. The most reliable methods of disruption depend on the use of expensive equipment (e.g. mechanical, sonic or ultrasonic disintegrators).

The addition of the protease and detergent to a sample of urine causes maximal exposure of intracellular bacterial enzymes present in that sample within 5 minutes at room temperature, and more rapidly at higher temperatures. The protease-detergent disrupting agent acts rapidly under near to physiological conditions of pH and temperature, and does not interfere with the detection of the catalase released by the treatment.

Initial studies summarized in said patent, have confirmed that disruption increases the sensitivity of catalase detection to the point where it can be used for rapid screening of urine. The results of an evaluation of a prototype strip test constructed on the above principle are given in Table II of said patent.

Further testing, however, of larger numbers of specimens (750 vs. 162 urine specimens) in a routine diagnostic laboratory yielded less satisfactory results with 35% false positives and a specificity of only 65% ("specificity" being defined as the number of true negatives divided by the sum of true negatives and false positives).

It was, therefore, realized that the preferred embodiment exemplified in said patent was not specific enough, as indicated by the unacceptably high rate of false positive results. Furthermore, it also became clear, following a careful and detailed study of said patent that there were no clues in that document that

2

would help in tracing the roots of the problem or suggest a solution.

It is, therefore, the main object of the present invention to provide an improved method for detecting the presence of cells in a urine sample which would yield unambiguous results. The specific requirements for such improved method being:

(a) Increased sensitivity, so as to ensure clear response of positive specimens;

(b) Elimination of irrelevant positive-characterizing foam-formation, so as to avoid spurious responses in negative specimens;

(c) Shortening the time of the test so that the positive-characterizing foam formation can be observed within 2-3 minutes after addition of the reagents to the sample.

(d) Increasing the stability of the foam formed so as to enable reliable reading of the results after about 1-2 hours or even more.

A further object of the invention is to provide an improved reagent mixture for carrying out the method of the invention.

Yet a further object of the invention is to provide an improved test kit for carrying out the method of the invention.

The above main object is achieved by the present invention which provides a method for testing a urine sample for the presence of bacterial or somatic cells, which comprises reacting said sample with a catalase-free alkaline protease enzyme and a detergent compatible therewith, so as to disrupt any such cells present in the sample and release active catalase therefrom, and detecting the presence of catalase by the formation of foam upon the addition of $H_2O_2$, characterized in that the reaction of said sample with said protease enzyme and said detergent is conducted in the presence of:

(a) a buffer providing for a pH of about 8.5 to about 11.0, preferably about 8.5 to about 10.0; and optionally

(b) one or more additional solutes;

the total concentration of components (a) and, if present, (b) being from about 0.025M to about 0.6M, preferably from about 0.2M to about 0.4M.

As disclosed in Israel Patent No. 36946, commercial preparations comprising proteases and detergents compatible therewith, in particular protease enzyme-enriched washing powders, can be most suitably be utilized for disrupting the cells . A suitable catalase-free alkaline protease enzyme enriched washing powder is the one marketed under the name BIOMAT® by Witco Chemical Ltd., P. O. Box 975, Haifa, Israel.

Preferably said buffer is Tris, present at a concentration of preferably about 20 mM, and said additional solute is $K_2HPO_4$, present at a concentration of preferably 40-70 mM. Other suitable buffers are phosphate salts and borate salts.

Other highly soluble inorganic salts (e.g. ammonium sulfate, sodium chloride, potassium chloride, magnesium chloride etc.) as well as soluble organic compounds (e.g. ureas, polyols, monosaccharides, sucrose etc.) can be used as said additional solute at sufficiently high concentrations in addition to said buffers to expel dissolved oxygen.

The main improvements of the method of the present invention as compared to that of Israel Patent No. 36946 are the selection of a pH range of about 8.5 to about 11.0 and the use of the buffer and the optional additional solute or solutes in the above specified total concentrations whereby said buffer and said additional solute (or solutes) decrease the solubility of oxygen released into said solution by the reaction of the catalase with the $H_2O_2$, with consequent increase of foam generation.

Thus, it has now been found in accordance with the present invention that buffering with 0.01M phosphate, as taught in the examples of said patent, is inadequate and the resulting pH (after mixing with the urine) is too low for effective cell disruption and a reaction of the released catalase with $H_2O_2$. The buffering must ensure a pH within the range of pH 8.5-11.0 (preferably, pH 8.5 to 10.0) for optimal cell disruption and a reaction of the released catalase with $H_2O_2$. In that range of pH the stability and activity of the catalase released is not materially impaired. Furthermore, since in accordance with the present invention at least twice as much and preferably even more than 5 times as much buffer is added alone or in combination with one or more additional solutes capable of reducing the solubility of oxygen in urine, the sensitivity of the test is increased even more due to the fact that all oxygen generated by the catalase reaction enters into the foam generating process.

Israel Patent No. 36496 states that the method described and claimed therein permits the detection of $50-100 \times 10^3$ bacteria in 1 ml of sample. As shown by the Examples hereinbelow, the improved method of the present invention exhibits a significantly higher sensitivity, by about one order of magnitude as compared to the method of said Patent. This increased sensitivity meets the requirements of physicians and medical institutions.

3

In another aspect of the present invention, there is provided a reagent mixture for carrying out the method of the invention, which mixture comprises one or more catalase-free alkaline protease enzymes, a detergent compatible with said enzyme (or enzymes), a buffer and optionally one or more additional solutes, wherein the concentrations of the buffer and the additional solute (or solutes), when present, are such that when a predetermined amount of said reagent mixture is mixed with a predetermined volume of a urine sample and a predetermined volume of an aqueous $H_2O_2$, the resultant solution will have a pH of about 8.5 to about 11.0, preferably about 8.5 to about 10.0, and a total concentration of said buffer and said additional solute (or solutes), when present, of about 0.025M to about 0.6M, preferably about 0.2M to about 0.4M.

The reagent mixture according to this aspect of the invention may preferably comprise also a dye which serves as a dark background for a clearer detection of the pale foam.

The reagent mixture according to the invention is preferably in dry form, i.e. in the form of a powder which, when a predetermined amount thereof is mixed with a predetermined volume of a urine sample and a predetermined vulume of an aqueous $H_2O_2$ solution, will provide for a pH of about 8.5 to about 11.0, preferably about 8.5 to about 10.0, in the resultant solution and a total concentration of buffer and additional solute (or solutes), when present, of about 0.025M to about 0.6M, preferably from about 0.2M to about 0.4M.

Alternatively, the reagent mixture according to the invention may be in the form of an aqueous solution wherein the concentrations of the buffer and the additional solute (or solutes), when present, are such that when a predetermined volume of said solution is mixed with a predetermined volume of a urine sample and a predetermined volume of an aqueous $H_2O_2$ solution, the resultant solution will have a pH of about 8.5 to about 11.0, preferably about 8.5 to about 10.0, and a total concentration of said buffer and said additional solute (or solutes), when present, from about 0.025M to about 0.6M, preferably from about 0.2M to about 0.4M. This aqueous solution may further comprise one or more preserving agents and/or stabilizers in order to prolong its shelf life.

In yet another aspect of the invention there is provided a test kit for carrying out the method of the invention, the kit comprising a packaged reagent mixture as described above, either in the form of an aqueous solution, or preferably, in the form of a dry powder.

The test kit according to the invention may optionally comprise also a packaged aqueous $H_2O_2$ solution and, optionally, also a plurality of test tubes suitably graduated so as to enable the addition thereto of desired volumes of urine, an aqueous $H_2O_2$ solution and possibly also an aqueous reagent solution as described above.

In accordance with a most preferred embodiment, the test kit of the invention comprises a plurality of test tubes suitably graduated so as to enable the addition thereto of a predetermined volume of urine and a predetermined volume of an aqueous $H_2O_2$ solution, each of said test tubes containing a reagent mixture in powder form packaged therein, in an amount such that when mixed with said predetermined volume of urine and said predetermined volume of an aqueous $H_2O_2$ solution, the resultant solution will have a pH of about 8.5 to 11.0, preferably about 8.5 to about 10.0, and a total concentration of buffer and additional solute (or solutes), when present, of about 0.025M to 0.6M, preferably from 0.2M to about 0.4M.

The invention will now be described in more detail in the following non-limiting examples. It is not intended to limit the invention to these exemplified embodiments. On the contrary, it is intended to cover all alternatives, modifications and equivalents as may be included within the scope of the invention as defined in the appended claims.

## EXAMPLE 1

## PREPARATION OF REAGENTS

### MATERIALS

### Test Tubes

Tris buffer, purchased from "Serva" or from "Sigma" (U.S.A.) (Trisma).
Sodium borate, marketed by Merck (Germany)
Sodium dodecyl sulfate (SDS) marketed by "Sigma" (U.S.A.).
Giemsa Stain and Hydrogen Peroxide (30%) products of BDH Chemicals Ltd.

BM powder (BIOMAT)*, marketed by WITCO Chemical Ltd., P.O. Box 975, Haifa 31009.
Subtilysin marketed by "Sigma" (U.S.A.).

METHOD A

a) A 3M solution (a) of Tris in distilled water (pH 9.0) was prepared.
b) The Giemsa stain was diluted 1:25 in distilled water to form solution (b).
c) The Tris solution (a) and the Giemsa stain solution (b) were mixed in a 1:1 ratio.
d) BM powder (BIOMAT) was ground together with an equal weight of $K_2HPO_4$ and the resulting powder mix stored at room temperature.

| METHOD B (dry reagent mixture) | |
| --- | --- |
| BM powder (BIOMAT) | 40 g |
| Tris buffer | 40 g |
| Giemsa Stain | 1 g |
| $K_2HPO_4$ (optional) | (40 g) |
| Total weight | 81 g (121 g) |

The above listed reagents are charged into a ball mill and ground therein for about 30-45 seconds.

| METHOD C (dry reagent mixture) | |
| --- | --- |
| Sodium borate | 1.9 g |
| Sodium dodecyl sulfate | 1.0 g |
| Sodium chloride | 6.0 g |
| Tris buffer ("Trisma") | 4.5 g |
| Giemsa Stain | 0.1 g |
| Subtilisin | 0.0675 g |
| Total weight | 13.5675 g |

The above listed reagents were mixed together and ground in a ball mill as described in "Method B" above.

**EXAMPLE 2**

II. Testing of Urine with reagents according to "Method A" in Example 1

Procedure:

a) 15 mg of the powder mix (d) prepared as in Example 1 "Method A", were placed at the bottom of a test tube.
b) 0.05 ml of the Tris and Giemsa solution (1:1) prepared as in Example 1 "Method A", were added.
c) 1 ml of urine was added.
d) 0.20 ml of $H_2O_2$ (6%) was added to start the reaction i.e. the development of foam.
e) When a pronounced ring of foam was observed after 5 minutes, the urine was scored as positive. The foam formed was stable for 1-2 hours, or sometimes more.

Results:

Almost 1500 clinical urine samples were tested, both in accordance with the above procedure and by conventional clinical laboratory methods (e.g. bacterial colony counting). The results are summarized in the following Table I.

*(Enzyme-enriched washing powder. The enzyme is an alkaline protease produced by a Carlsberg Strain of Bacillus subtilis and marketed under the name of Alkalase or Subtilisin.)

## TABLE I

| | Series I | Series II |
|---|---|---|
| | (Dec. 85-Feb. 86) | (March 1986) |
| Total No. | 925 | 571 |
| True positives | 366 | 240 |
| False positives | 82 | 50 |
| True negatives | 467 | 273 |
| False negatives | 10 | 8 |
| Sensitivity[a] | 97.3 | 96.7 |
| Specificity[b] | 85.0 | 84.5 |
| Pos. predicted value[c] | 81.6 | 82.7 |
| Neg. predicted value[d] | 97.9 | 97.1 |

(a) $\dfrac{\text{true positives}}{\text{true positives + false negatives}} \times 100$

(b) $\dfrac{\text{true negatives}}{\text{true negatives + false positives}} \times 100$

(c) $\dfrac{\text{true positives}}{\text{true positives + false positives}} \times 100$

(d) $\dfrac{\text{true negatives}}{\text{true negatives + false negatives}} \times 100$

The visual determination of the positive reaction is facilitated by:

1) the detergent, which participates in the disruption of the cells, and is also suitable for providing foam layer which forms when oxygen is released from hydrogen peroxide by the action of catalase; and

2) the dye (e.g. Giemsa Stain) which provides a dark background against which the foaming (Positive reaction) is clearly visible.

The above procedure therefore satisfies the requirements for a rapid and reliable test for bacterial or somatic cells in urine. Furthermore, the addition of both solid $K_2HPO_4$ and Tris serves a dual purpose: They act as a buffer, so that all urine specimens are examined at the optimal pH, and they also serve to decrease the solubility of the oxygen released by the catalase reaction with $H_2O_2$, thereby enhancing the sensitivity of the test.

## EXAMPLE 3

### Comparison of the sensitivity of the method of Example 2 according to the present invention with that of the method of Israel Patent No. 36469

Bacteria-free urine ("Control" in Table II) was seeded with Staphylococci at a concentration of $5\times10^7$ bacteria/ml. A series of dilutions were prepared with concentrations as specified in the following Table II.

Four samples of each dilution were tested in accordance with the procedure described in Example 2 herein ("New method" in Table II) and 4 samples in accordance with the procedure described in Example 3 of Israel Patent No. 36469 ("Old method" in Table II).

The entire series of tests was repeated independently. The results (average of 2x4 tests by each method with each dilution) are summarized in the following Table II.

Table II

| Dilution | New Method | Old Method |
|----------|------------|------------|
| Control | - | - |
| 1:10 | + | + |
| 1:20 | + | + |
| 1:40 | + | + |
| 1:80 | + | + |
| 1:160 | + | - |
| 1:320 | + | - |
| 1:640 | + | - |
| 1:1280 | - | - |
| - no foam evolved, negative result. | | |
| + foam evolved, positive result. | | |

## EXAMPLE 4

### Testing of Urine with dry reagent mixture according to "Method C" in Example 1

Procedure:

a) 95±5 mg of the dry reagent mixture prepared as in "Method C" in Example 1 were introduced into a test tube containing 2 glass beads having an average diameter of 3 mm.
b) 1.5 ml of urine and 4 drops of $H_2O_2$ (10%) were added.
c) The test tube was gently shaken and allowed to stnad for 1-2 minutes.
d) When a pronounced ring of foam was observed around the periphery of the surface of the liquid, the urine was scored as positive. In the absence of foam the urine was scored as negative.
The foam formed was stable for 1-2 hours.

Results:

33 urine samples obtained from patients in the Kaplan Hospital, Rehovoth, Israel were tested both in accordance with the above procedure and by conventional clinical laboratory methods (e.g. bacterial colony counting). The results are summarized in the following Table III.

## TABLE III

| Sample No. | "New Method"[a] | Culture[b] | Dipstick[c] | Microscopical |
|---|---|---|---|---|
| 1 | + | + | + nitr. | |
| 2 | + | - | + nitr. | |
| 3 | + | - | + eryth. | |
| 4 | -+ | -+ ($<10^4$) | - | |
| 5 | -+ | -+ | - | |
| 6 | - | - | - | |
| 7 | -+ | + ($10^4$) | - | |
| 8 | - | - | - | |
| 9 | - | - | - | |
| 10 | - | - | - | |
| 11 | + | - | - | + |
| 12 | + | + | - | |
| 13 | + | + | + | |
| 14 | + | - | - | + leuko. |
| 15 | - | - | - | |
| 16 | - | - | - | |
| 17 | + | -+ | + leuko | |
| 18 | ⏐ | ⏐ | - | |
| 19 | + | + | + leuko | |
| 20 | + | - | + eryth | |

## TABLE III (continued)

| Sample No. | "New Method"[a] | Culture[b] | Dipstick[c] | Microscopical |
|---|---|---|---|---|
| 21 | + | + | - | + leuko |
| 22 | + | + | + nitr | |
| 23 | --+ | - | + leuko | |
| 24 | - | - | -- | |
| 25 | - | - | - | |
| 26 | + | + | - | |
| 27 | + | + | + | |
| 28 | --+ | + ($10^4$) | + | + leuko |
| 29 | + | + | - | |
| 30 | --+ | -- | - | - |
| 31 | + | + | + leuko | |
| 32 | + | - | + eryth | + eryth leuko |
| 33 | -++ | - | + eryth | + eryth |

(a) By the procedure of Example 4.

(b) Conventional test procedure by culturing on the following culture media:

C.L.E.D. Medium (Diagnostics Pasteur, France)

MacConkey agar (Diagnostics Pasteur, France)

Blood agar base (Diagnostics Pasteur, France)

(c) Dipstic "Urotron RL9" (Boehringer Mannheim, Germany)

## Claims

1. A method for testing a urine sample for the presence of bacterial or somatic cells, which comprises reacting said sample with a catalase-free alkaline protease enzyme and a detergent compatible therewith, so as to disrupt any such cells present in the sample and release active catalase therefrom, and detecting the presence of catalase by the formation of foam upon the addition of $H_2O_2$, characterized in that the reaction of said sample with said protease enzyme and said detergent is conducted in the presence of:

(a) a buffer providing for a pH of 8.5 to 11.0; and optionally
(b) one or more additional solutes;
the total concentration of components (a) and, if present, (b) being from 0.025M to 0.6M.

2. A method according to Claim 1, wherein the buffer (a) provides for a pH of 8.5 to 10.0.

3. A method according to Claim 1 or 2 wherein the total concentration of components (a) and if present, (b) is from 0.2M to 0.4M.

4. A method according to any one of Claims 1 to 3 wherein said buffer is selected from Tris, phosphate salts, borate salts and combinations thereof.

5. A method according to any one of Claims 1 to 4, wherein said additional solute is selected from inorganic salts, organic polyols, monosaccharides, disaccharades and urea.

6. A method according to Claim 5, wherein said additional solute is selected from phosphate, sulfate and chloride salts.

7. A method according to Claim 6, wherein said buffer is Tris and said additional solute is $K_2HPO_4$.

8. A method according to any one of Claims 1 to 7, wherein a dye is added to the urine sample so as to serve as a dark background in order to enable an easy observation of the pale foam.

9. A method according to Claim 8, wherein the dye is Giemsa stain.

10. A reagent mixture for carrying out the method of Claim 1 which comprises one or more catalase-free alkaline protease enzymes, a detergent compatible with said enzyme (or enzymes), a buffer and optionally one or more additional solutes, wherein the concentrations of the buffer and, if present, the additional solute (or solutes), are such that when a predetermined amount of said reagent mixture is mixed with a predetermined volume of a urine sample and a predetermined volume of an aqueous $H_2O_2$ solution, the resultant solution will have a pH of 8.5 to 11.0 and a total concentration of said buffer and said additional solute (or solutes), when present, of 0.025M to 0.6M.

11. A reagent mixture according to Claim 10, wherein the concentration of the buffer is such as to provide in the resulting solution a pH of 8.5 to 10.0.

12. A reagent mixture according to Claim 10 or 11, wherein the total concentration of components (a) and, if present, (b) is such as to provide in the resulting solution a pH of 8.5 to 10.0 total concentration of 0.2M to 0.4M.

13. A reagent mixture according to any one of Claims 10 to 12 in dry powder form.

14. A reagent mixture according to any one of Claims 10 to 12 in the form of an aqueous solution.

15. A reagent mixture according to any one of Claims 10 to 14 further comprising a dye.

16. A reagent mixture according to any one of Claims 10 to 15, wherein said buffer is selected from Tris, phosphate salts, borate salts and combinations thereof.

17. A reagent mixture according to any one of Claims 10 to 16, wherein said additional solute is selected from inorganic salts, organic polyols, monosaccharides, disaccharides and urea.

18. A reagent mixture according to Claim 17, wherein said additional solute is selected from phosphate, sulfate and chloride salts.

19. A reagent mixture according to Claims 16 and 18, wherein said buffer is Tris and the additional solute is $K_2HPO_4$.

**20.** A test kit for carrying out the method of Claim 1, comprising a packaged reagent mixture according to any one of Claims 10 to 19 and optionally a packaged aqueous $H_2O_2$ solution.

**21.** A test kit for carrying out the method of Claim 1, comprising a plurality of test tubes suitably graduated so as to enable the addition thereto of a predetermined volume of urine and a predetermined volume of an aqueous $H_2O_2$ solution, each of said test tubes containing a reagent mixture according to Claim 13 packaged therein, in an amount such that when mixed with said predetermined volume of urine and said predetermined volume of an aqueous $H_2O_2$ solution, the resultant solution will have a pH of 8.5 to 11.0 and a total concentration of buffer and, if present, additional solute (or solutes), of 0.025M to 0.6M.

**22.** A test kit according to Claim 20, further comprising a plurality of test tubes suitably graduated so as to enable the addition thereto of predetermined volumes of urine, an aqueous reagent solution according to Claim 14 and an aqueous $H_2O_2$ solution.

**Patentansprüche**

**1.** Verfahren zum Testen einer Urinprobe auf die Anwesenheit von Bakterien- oder Somazellen, welches umfaßt, daß besagte Probe mit einem katalasefreien, alkalischen Protease-Enzym und einem damit verträglichen Tensid zur Reaktion gebracht wird, um alle solche Zellen, die in der Probe vorhanden sind, aufzubrechen, und aktive Katalayse daraus freizusetzen, und daß die Anwesenheit von Katalase durch die Bildung von Schaum bei der Zugabe von $H_2O_2$ nachgewiesen wird, dadurch gekennzeichnet, daß die Reaktion besagter Probe mit besagtem Protease-Enzym und besagtem Tensid durchgeführt wird in der Gegenwart von:
   (a) einem Puffer, der einen pH von 8,5 bis 11,0 bereitstellt; und fakultativ
   (b) einer oder mehreren zusätzlichen Beimengungen;
wobei die Gesamtkonzentration der Komponenten (a) und, falls vorhanden, (b) von 0,025 M bis 0,6 M beträgt.

**2.** Verfahren nach Anspruch 1, dadurch gekennzeichnet, daß der Puffer (a) einen pH von 8,5 bis 10,0 bereitstellt.

**3.** Verfahren nach Anspruch 1 oder 2, dadurch gekennzeichnet, daß die Gesamtkonzentration der Komponenten (a) und, falls vorhanden, (b) von 0,2 M bis 0,4 M beträgt.

**4.** Verfahren nach einem der Ansprüche 1 bis 3, dadurch gekennzeichnet, daß besagter Puffer ausgewählt ist aus Tris, Phosphatsalzen, Boratsalzen und Kombinationen derselben.

**5.** Verfahren nach einem der Ansprüche 1 bis 4, dadurch gekennzeichnet, daß besagte zusätzliche Beimengung ausgewählt ist aus anorganischen Salzen, organischen Polyolen, Monosacchariden, Disacchariden und Harnstoff.

**6.** Verfahren nach Anspruch 5, dadurch gekennzeichnet, daß besagte zusätzliche Beimengung ausgewählt ist aus Phosphat-, Sulfat- und Chloridsalzen.

**7.** Verfahren nach Anspruch 6, dadurch gekennzeichnet, daß besagter Puffer Tris ist und daß besagte zusätzliche Beimengung $K_2HPO_4$ ist.

**8.** Verfahren nach einem der Ansprüche 1 bis 7, dadurch gekennzeichnet, daß ein Farbstoff zur Urinprobe zugesetzt wird, um als ein dunkler Hintergrund zu dienen, um eine leichte Beobachtung des blassen Schaums zu ermöglichen.

**9.** Verfahren nach Anspruch 8, dadurch gekennzeichnet, daß der Farbstoff ein Giemsa-Färbemittel ist.

**10.** Reagenzmischung zur Durchführung des Verfahrens nach Anspruch 1, die ein oder mehrere katalasefreie, alkalische Protease-Enzyme, ein mit besagtem Enzym (oder Enzymen) verträgliches Tensid, einen Puffer und fakultativ eine oder mehrere zusätzliche Beimengungen umfaßt, wobei die Konzentrationen des Puffers und, falls vorhanden, der zusätzlichen Beimengung (oder Beimengungen) derart sind, daß, wenn eine vorbestimmte Menge besagter Reagenzmischung mit einem vorbestimmten Volumen einer Urinprobe und einem vorbestimmten Volumen einer wäßrigen $H_2O_2$-Lösung vermischt wird, die resultierende Lösung einen pH von 8,5 bis 11,0 und eine Gesamtkonzentration an besagtem Puffer und besagter zusätzlichen Beimischung (oder Beimischungen), wenn vorhanden, von 0,025 M bis 0,6 M aufweisen wird.

11. Reagenzmischung nach Anspruch 10, dadurch gekennzeichnet, daß die Konzentration des Puffers derart ist, daß in der resultierenden Lösung ein pH von 8,5 bis 10,0 bereitgestellt wird.

12. Reagenzmischung nach Anspruch 10 oder 11, dadurch gekennzeichnet, daß die Gesamtkonzentration der Komponenten (a) und, falls vorhanden, (b) derart ist, daß in der resultierenden Lösung ein pH von 8,5 bis 10,0, Gesamtkonzentration von 0,2 M bis 0,4 M bereitgestellt wird.

13. Reagenzmischung nach einem der Ansprüche 10 bis 12 in Trockenpulverform.

14. Reagenzmischung nach einem der Ansprüche 10 bis 12 in der Form einer wäßrigen Lösung.

15. Reagenzmischung nach einem der Ansprüche 10 bis 14, die außerdem einen Farbstoff umfaßt.

16. Reagenzmischung nach einem der Ansprüche 10 bis 15, dadurch gekennzeichnet, daß besagter Puffer ausgewählt ist aus Tris, Phosphatsalzen, Boratsalzen und Kombinationen derselben.

17. Reagenzmischung nach einem der Ansprüche 10 bis 16, dadurch gekennzeichnet, daß besagte zusätzliche Beimengung ausgewählt ist aus anorganischen Salzen, organischen Polyolen, Monosacchariden, Disacchariden und Harnstoff.

18. Reagenzmischung nach Anspruch 17, dadurch gekennzeichnet, daß besagte zusätzliche Beimengung ausgewählt ist aus Phosphat-, Sulfat- und Chloridsalzen.

19. Reagenzmischung nach den Ansprüchen 16 und 18, dadurch gekennzeichnet, daß besagter Puffer Tris ist und daß die zusätzliche Beimengung $K_2HPO_4$ ist.

20. Test-Kit zur Durchführung des Verfahrens nach Anspruch 1, welcher eine abgepackte Reagenzmischung gemäß einem der Ansprüche 10 bis 19 und fakultativ eine abgepackte wäßrige $H_2O_2$-Lösung umfaßt.

21. Test-Kit zur Durchführung des Verfahrens nach Anspruch 1, welcher eine Mehrzahl von Teströhrchen umfaßt, die in geeigneter Weise mit Meßeinteilung versehen sind, um dorthinein die Zugabe eines vorbestimmten Volumens Harnstoffs und eines vorbestimmten Volumens einer wäßrigen $H_2O_2$-Lösung zu ermöglichen, wobei jedes besagter Teströhrchen eine Reagenzmischung gemäß Anspruch 13 enthält, die darin abgepackt ist, in einer solchen Menge, daß, wenn sie mit besagtem vorbestimmten Volumen Urin und besagtem vorbestimmten Volumen einer wäßrigen $H_2O_2$-Lösung vermischt wird, die resultierende Lösung einen pH von 8,5 bis 11,0 und eine Gesamtkonzentration ann Puffer und, falls vorhanden, zusätzlicher Beimengung (oder Beimengungen), von 0,025 M bis 0,6 M aufweisen wird.

22. Test-Kit nach Anspruch 20, der außerdem eine Mehrzahl von Teströhrchen umfaßt, die in geeigneter Weise mit Meßeinteilung versehen sind, um dorthinein die Zugabe vorbestimmter Volumenteile Harnstoff, einer wäßrigen Reagenzlösung nach Anspruch 14 und einer wäßrigen $H_2O_2$-Lösung zu ermöglichen.

## Revendications

1. Procédé pour tester dans un échantillon d'urine la présence de cellules bactériennes ou somatiques, dans lequel on fait réagir ledit échantillon avec une enzyme protéase alcaline dépourvue de catalase et un détergent compatible avec elle, de manière à rompre toutes cellules de ce type éventuellement présentes dans l'échantillon et à en libérer la catalase active, et l'on détecte la présence de catalase par la formation de mousse lors de l'addition d'$H_2O_2$ , caractérisé en ce que la réaction dudit échantillon avec ladite enzyme protéase et ledit détergent est conduite en présence de:
   (a) un tampon assurant un pH de 8,5 à 11,0; et facultativement

EP 0 369 041 B1

(b) un ou plusieurs solutés additionnels;
la concentration totale de composants (a) et, s'il y en a, de (b) étant de 0,025 M à 0,6 M.

2. Procédé selon la revendication 1, dans lequel le tampon (a) assure un pH de 8,5 à 10,0.

3. Procédé selon la revendication 1 ou 2 dans lequel la concentration totale de composants (a) et, s'il y en a, de (b), est de 0,2 M à 0,4 M.

4. Procédé selon l'une quelconque des revendications 1 à 3 dans lequel ledit tampon est choisi parmi le trométamol, les sels de phosphate, les sels de borate et leurs combinaisons.

5. Procédé selon l'une quelconque des revendications 1 à 4, dans lequel ledit soluté additionnel est choisi parmi les sels inorganiques, les polyols organiques, les monosaccharides, les disaccharides et l'urée.

6. Procédé selon la revendication 5, dans lequel ledit soluté additionnel est choisi parmi les sels de phosphate, sulfate et chlorure.

7. Procédé selon la revendication 6, dans lequel ledit tampon est le trométamol et ledit soluté additionnel est $K_2HPO_4$.

8. Procédé selon l'une quelconque des revendications 1 à 7, dans lequel on ajoute un colorant à l'échantillon d'urine de manière à servir de fond foncé pour permettre d'observer facilement la mousse pâle.

9. Procédé selon la revendication 8, dans lequel le colorant est la coloration de Giemsa.

10. Mélange réactif pour réaliser le procédé de la revendication 1 qui comprend une ou plusieurs enzymes de type protéase alcaline dépourvue de catalase, un détergent compatible avec le(s)dit(s) enzyme(s), un tampon et le cas échéant un ou plusieurs solutés additionnels, où les concentrations du tampon et, s'il y en a, du(des) soluté(s) additionnel(s), sont telles que lorsqu'on mélange une quantité prédéterminée dudit mélange réactif avec un volume prédéterminé d'un échantillon d'urine et un volume prédéterminé d'une solution aqueuse d'$H_2O_2$ , la solution résultante ait un pH de 8,5 à 11,0 et une concentration totale dudit tampon et dudit(desdits) soluté(s) additionnel(s), lorsqu'il y en a, de 0,025 M à 0,6 M.

11. Mélange réactif selon la revendication 10, dans lequel la concentration du tampon est telle qu'elle fournisse dans la solution résultante un pH de 8,5 à 10,0.

12. Mélange réactif selon la revendication 10 ou 11, dans lequel la concentration totale des composants (a) et, s'il y en a, (b) est telle qu'elle fournisse dans la solution résultante un pH de 8,5 à 10,0 pour une concentration totale de 0,2 M à 0,4 M.

13. Mélange réactif selon l'une quelconque des revendications 10 à 12 sous forme de poudre sèche.

14. Mélange réactif selon l'une quelconque des revendications 10 à 12 sous la forme d'une solution aqueuse.

15. Mélange réactif selon l'une quelconque des revendications 10 à 14 comprenant en outre un colorant.

16. Mélange réactif selon l'une quelconque des revendications 10 à 15, dans lequel ledit tampon est choisi parmi le trométamol, les sels de phosphate, les sels de borate et leurs combinaisons.

17. Mélange réactif selon l'une quelconque des revendications 10 à 16, dans lequel ledit soluté additionnel est choisi parmi les sels inorganiques, les polyols organiques, les monosaccharides, les disaccharides et l'urée.

18. Mélange réactif selon la revendication 17, dans lequel ledit soluté additionnel est choisi parmi les sels de phosphate, de sulfate de de chlorure.

13

**19.** Mélange réactif selon les revendications 16 et 18, dans lequel ledit tampon est le trométamol et le soluté additionnel est $K_2HPO_4$.

**20.** Nécessaire expérimental pour réaliser le procédé de la revendication 1, comprenant un mélange réactif conditionné selon l'une quelconque des revendications 10 à 19 et si on le désire une solution aqueuse d'$H_2O_2$ conditionnée.

**21.** Nécessaire expérimental pour réaliser le procédé de la revendication 1, comprenant plusieurs tubes à essais gradués de façon appropriée de manière à permettre d'y ajouter un volume prédéterminé d'urine et un volume prédéterminé d'une solution aqueuse d'$H_2O_2$ , chacun desdits tubes à essais contenant un mélange réactif selon la revendication 13 qui y est conditionné, en une quantité telle que lorsqu'on mélange avec ledit volume prédéterminé d'urine et ledit volume prédéterminé d'une solution aqueuse d'$H_2O_2$ , la solution résultante ait un pH de 8,5 à 11,0 et une concentration totale de tampon et, s'il y en a, de soluté(s) additionnel(s), de 0,025 M à 0,6 M.

**22.** Nécessaire expérimental selon la revendication 20, comprenant en outre plusieurs tubes à essais gradués de façon appropriée de manière à permettre d'y ajouter des volumes prédéterminés d'urine, d'une solution aqueuse de réactif selon la revendication 14 et d'une solution aqueuse d'$H_2O_2$.